(19) 

Europäisches
Patentamt

European
Patent Office

Office européen
des brevets

(11) **EP 1 985 233 B1**

(12) **FASCICULE DE BREVET EUROPEEN**

(45) Date de publication et mention
de la délivrance du brevet:
**18.07.2018 Bulletin 2018/29**

(51) Int Cl.:
***G01C 21/16*** *(2006.01)*

(21) Numéro de dépôt: **08290390.7**

(22) Date de dépôt: **21.04.2008**

(54) **Procédé et dispositif de détection d'un axe de rotation sensiblement invariant**

Verfahren und Vorrichtung zur Erfassung einer extrem unveränderlichen Drehachse

Method and device for detecting a substantially invariant axis of rotation

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **25.04.2007 FR 0754693**

(43) Date de publication de la demande:
**29.10.2008 Bulletin 2008/44**

(60) Demande divisionnaire:
**18173359.3**

(73) Titulaire: **Commissariat à l'Énergie Atomique
et aux Énergies Alternatives
75015 Paris (FR)**

(72) Inventeurs:
• **Godin, Christelle
38190 Brignoud (FR)**
• **Bonnet, Stéphane
38170 Seyssinet (FR)**

• **Barraud, Alain
38190 Froges (FR)**
• **Lesecq, Suzanne
38190 Froges (FR)**

(74) Mandataire: **Santarelli
49, avenue des Champs-Elysées
75008 Paris (FR)**

(56) Documents cités:
**EP-A2- 1 965 169     US-A- 3 731 521
US-A- 5 105 152**

• **CHUNLEI RUI ET AL: "Nonlinear Attitude and
Shape Control of Spacecraft with Articulated
Appendages and Reaction Wheels" IEEE
TRANSACTIONS ON AUTOMATIC CONTROL,
IEEE SERVICE CENTER, LOS ALAMITOS, CA, US,
vol. 45, no. 8, août 2000 (2000-08), XP011001383
ISSN: 0018-9286**

Printed by Jouve, 75001 PARIS (FR)

**Description**

La présente invention concerne un procédé de détection d'un axe de rotation sensiblement invariant d'un mouvement d'un solide.

Elle concerne également un procédé d'estimation d'un mouvement d'un solide.

Corrélativement, la présente invention concerne un dispositif de détection d'un axe de rotation sensiblement invariant d'un mouvement d'un solide.

De manière générale, la présente invention concerne le domaine de la capture de mouvements. Les domaines d'application sont variés et couvrent en particulier le domaine biomédical, pour l'observation des personnes, le domaine sportif, pour l'analyse d'un mouvement, d'un sportif lui-même ou de son équipement (raquettes, balles ...). Elle s'applique également au domaine de l'automobile ou de la robotique, ainsi qu'à la réalité virtuelle, et de manière générale à toute application comportant un corps mobile dont on cherche à déterminer ou à observer le mouvement.

On connaît en particulier des systèmes inertiels constitués d'un ou plusieurs capteurs choisis notamment parmi des accéléromètres, des magnétomètres ou des gyromètres, ayant un ou plusieurs axes de sensibilité.

Ces systèmes inertiels présentent l'avantage d'être autonomes et de ne pas requérir d'équipement préalable de l'environnement dans lequel on observe le mouvement d'un mobile.

On connaît en particulier un dispositif de capture de mouvement de rotation d'un solide tel que décrit dans le document FR 2 838 185.

Lors de l'observation d'un mobile en mouvement, le principe consiste à rechercher les grandeurs représentatives du déplacement et de l'orientation du mobile, ou encore de sa vitesse et vitesse de rotation ou de son accélération et accélération angulaire.

En pratique, les capteurs sont disposés sur le mobile observé et on cherche à déterminer un mouvement ayant potentiellement six degrés de liberté, c'est-à-dire trois degrés de liberté correspondant à l'orientation du solide dans l'espace et trois degrés de liberté correspondant à la position du mobile

La résolution des systèmes d'équations pour rechercher les grandeurs représentatives du mouvement d'un solide est généralement complexe et nécessite des systèmes de résolution dans lesquels certaines contraintes sont connues par avance.

On connaît par ailleurs dans le document US 3 731 521 un système permettant, à partir de la réponse de deux clusters de gyromètres et de leur comparaison, de déterminer la défaillance d'un gyromètre dans un système de navigation.

La présente invention a pour but de résoudre les inconvénients des systèmes d'estimation de mouvement antérieurs et de proposer un procédé particulièrement bien adapté au traitement du mouvement d'un mobile ayant un axe de rotation sensiblement invariant.

Selon un premier aspect, la présente invention vise un procédé de détection d'un axe de rotation sensiblement invariant d'un mouvement d'un mobile selon la revendication 1.

En effet, la demanderesse a constaté qu'en déterminant l'axe de rotation sensiblement invariant parmi les mesures obtenues en sortie d'un même capteur, il est possible de déterminer par là même l'axe de rotation sensiblement constant du mouvement du mobile équipé de ce capteur.

L'estimation de l'axe de rotation dans l'espace des mesures physiques nécessite de disposer d'au moins trois mesures différentes à des instants différents et selon les trois axes de sensibilité du capteur, pour permettre la détermination des coordonnées de l'axe de rotation estimé.

En pratique, le procédé de détection comprend en outre les étapes suivantes :

- comparaison de la valeur de l'indicateur avec une valeur de seuil prédéterminée ; et
- validation de l'axe de rotation estimé comme axe de rotation sensiblement invariant du mouvement lorsque la valeur de l'indicateur est inférieure à la valeur de seuil prédéterminée, avantageusement égale à la valeur du bruit des mesures physiques dudit capteur.

Ainsi, grâce à un indicateur de variations de l'axe de rotation estimé, il est possible d'obtenir une indication sur les variations de l'axe de rotation du mouvement. Si la valeur de cet indicateur est suffisamment faible, c'est-à-dire de l'ordre de la valeur du bruit des mesures physiques du capteur observé, on peut considérer que le mouvement correspond bien à une rotation autour d'un axe sensiblement invariant. Il est ainsi possible de valider l'axe de rotation estimé dans l'espace des mesures physiques comme axe de rotation sensiblement invariant du mouvement du mobile.

En particulier, lorsque l'un des coefficients de la combinaison est égal à 0, il est possible d'utiliser l'un ou l'autre capteur (magnétomètre ou accéléromètre) permettant d'obtenir l'estimation la plus fiable de l'axe de rotation sensiblement invariant.

En particulier, lorsque la rotation a lieu autour d'un axe du champ physique considéré (le champ de gravité pour les accéléromètres, le champ magnétique terrestre pour les magnétomètres), il est possible de déterminer l'axe de rotation invariant en utilisant l'autre capteur, dès lors que l'indicateur des variations de l'axe de rotation estimé au moyen des mesures physiques du capteur associé au champ physique invariant sera nécessairement mauvais, c'est-à-dire très supérieur à la valeur du bruit des mesures physiques obtenues par ce capteur.

Dans un mode de réalisation pratique de l'invention, le procédé de détection comporte en outre une étape de transformation des coordonnées de l'axe de rotation estimé dans le repère de mesure du capteur en coordonnées dans un repère géophysique, tel que dans un repère NED (acronyme pour *North-East-Down*). Un mode de réalisation de la présente invention concerne un procédé d'estimation d'un mouvement d'un mobile équipé d'au moins un magnétomètre à trois axes de sensibilité et un accéléromètre à trois axes de sensibilité, le mouvement comprenant une rotation autour d'un axe sensiblement invariant. Le procédé d'estimation comprend les étapes suivantes :

- détection d'un axe de rotation sensiblement invariant selon le procédé de détection conforme à l'invention ; et
- détermination de l'angle de rotation du mobile autour de l'axe de rotation sensiblement invariant.

Le procédé d'estimation peut comprendre en outre une étape de détermination de l'accélération du mobile.

Grâce au procédé de détection d'un axe de rotation sensiblement invariant conforme à l'invention, le procédé d'estimation du mouvement d'un mobile est facilité dès lors que le nombre d'inconnues à déterminer est limité à la détermination de l'angle de rotation, et éventuellement de l'accélération du mobile.

Après détection de l'axe de rotation invariant conformément à l'invention, il est possible de calculer ce mouvement en deux étapes, par la recherche tout d'abord de l'angle de rotation, puis le calcul de l'accélération du mobile.

Selon un troisième aspect, qui ne constitue pas l'invention, un procédé de calibration d'un capteur inertiel ou magnétique à trois axes de sensibilité permet de déterminer une matrice de rotation adaptée à transformer les coordonnées exprimées dans un repère de mesure du capteur en des coordonnées exprimées dans un repère prédéterminé solidaire d'un mobile portant le capteur.

Ce procédé de calibration comprend les étapes suivantes :

- acquisition d'au moins deux séries de mesures physiques par le capteur correspondant respectivement à la rotation du mobile selon au moins deux des trois axes du repère prédéterminé solidaire du mobile, chaque série de mesures physiques comprenant au moins trois échantillons à des instants différents ;
- détermination à partir de chacune des deux séries des coordonnées de chacun des axes de rotation correspondant à l'un desdits deux des trois axes du repère prédéterminé solidaire du mobile dans le repère des mesures physiques du capteur selon le procédé de détection conforme à l'invention ; et
- élaboration d'une matrice de rotation à partir des coordonnées déterminées desdits au moins deux des trois axes de rotation.

Ce procédé de calibration permet ensuite d'exprimer les mesures obtenues au niveau du capteur dans un repère prédéterminé lié au mobile observé, tel qu'un boîtier renfermant le capteur ou encore une partie anatomique d'un individu sur laquelle est positionné le capteur.

Enfin, la présente invention concerne selon un quatrième aspect un dispositif de détection d'un axe de rotation sensiblement invariant d'un mouvement d'un mobile équipé d'au moins un capteur inertiel ou magnétique à trois axes de sensibilité.

Ce dispositif de détection comprend des moyens adaptés à mettre en oeuvre le procédé de détection conforme à l'invention.

Ce dispositif de détection présente des caractéristiques et avantages analogues à ceux décrits précédemment en relation avec le procédé de détection.

D'autres particularités et avantages de l'invention apparaîtront dans la description ci-après.

Aux dessins annexés, donnés à titre d'exemple non limitatif :

- la figure 1 est un algorithme illustrant un procédé de détection d'un axe de rotation sensiblement invariant conforme à un premier mode de réalisation de l'invention ;
- la figure 2 est un schéma illustrant l'étape d'estimation d'un axe de rotation sensiblement invariant conformément à l'invention ;
- la figure 3 est un algorithme illustrant un procédé de détection d'un axe de rotation sensiblement invariant selon un second mode de réalisation de l'invention ;
- la figure 4 est un algorithme illustrant un procédé d'estimation d'un mouvement d'un mobile conformément à un mode de réalisation de l'invention ;
- la figure 5 est un schéma illustrant un dispositif de calibration, intégré dans un boîtier ;
- la figure 6 est un schéma illustrant un dispositif de calibration, dans lequel le capteur est positionné sur une partie anatomique ; et

- la figure 7 est un algorithme illustrant le procédé de calibration d'un capteur.

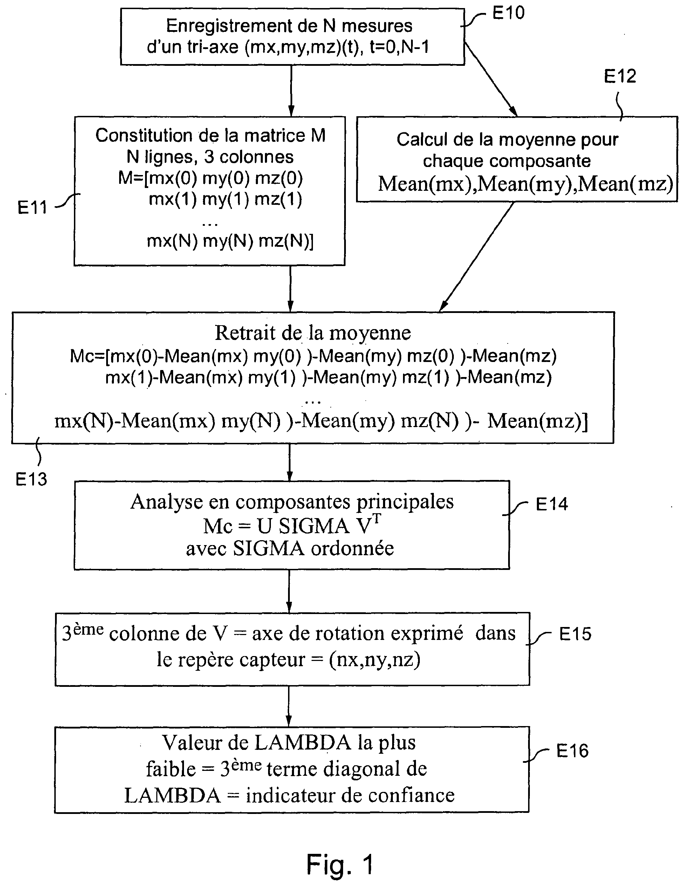

On va décrire tout d'abord en référence à la figure 1, un premier mode de réalisation d'un procédé de détection d'un axe de rotation sensiblement invariant d'un mouvement d'un mobile équipé d'un capteur à trois axes de sensibilité.

Ce procédé peut s'appliquer à l'étude du mouvement d'un mobile dans de nombreuses applications dès lors que le mouvement de ce mobile correspond à une rotation autour d'un axe fixe ou sensiblement invariant.

Il existe en effet de nombreuses applications dans lesquelles le mouvement du mobile observé correspond à une rotation autour d'un axe, avec en outre généralement des déplacements nuls selon cet axe de rotation de telle sorte que le mouvement est réalisé dans un plan et peut être considéré comme un mouvement planaire.

A titre d'exemple, un tel mouvement est observé pour un véhicule dans lequel le roulis et le tangage sont nuls. La rotation du véhicule correspond à une rotation autour d'un axe vertical fixe, correspondant à un mouvement de lacet. Généralement, l'accélération verticale est nulle de telle sorte que le mouvement du véhicule est un mouvement planaire.

De même, l'observation de mouvements des membres d'une personne (bras, jambes, ...) est réalisée dans un plan dès lors qu'il n'existe qu'un seul axe de rotation autour d'une articulation et un angle qui varie au cours du temps.

Le mouvement d'une roue qui roule sans glissement sur un plan et en ligne droite correspond également à un mouvement autour d'un axe de rotation, perpendiculaire au plan de la roue.

Le déplacement d'une souris d'ordinateur dans un plan autour d'un axe orthogonal à ce plan est également un mouvement planaire qui peut être observé dans le cadre de la présente invention.

De manière générale, le procédé de détection de l'axe de rotation sensiblement invariant d'un tel mouvement est mis en oeuvre à l'aide d'un dispositif de détection équipé d'au moins un capteur à trois axes de sensibilité, tel que par exemple un accéléromètre, un magnétomètre ou un gyromètre monté en tri-axe.

Dans son principe, le procédé de détection consiste à estimer un axe de rotation sensiblement invariant dans l'espace des mesures physiques obtenues par le capteur à trois axes de sensibilité.

Dans le mode de réalisation illustré à la figure 1, on considère que l'axe de rotation sensiblement invariant dans l'espace des mesures correspond à l'axe de plus faible variation des mesures pour le magnétomètre et l'accéléromètre et de plus grande variation pour le gyromètre.

Chaque mesure à un instant t obtenue en sortie d'un capteur à trois axes de sensibilité est considérée comme un point dans l'espace des mesures à trois dimensions.

L'axe invariant correspond ainsi à l'axe pour lequel les mesures présentent pour un magnétomètre ou un accéléromètre la plus faible variation sur cet axe, c'est-à-dire que l'écart-type des points projetés sur cet axe est le plus faible.

Dans le cas d'un gyromètre, ce sera l'axe de plus grande variation puisque le gyromètre mesure sur chacun de ses axes, la valeur de la rotation autour de cet axe.

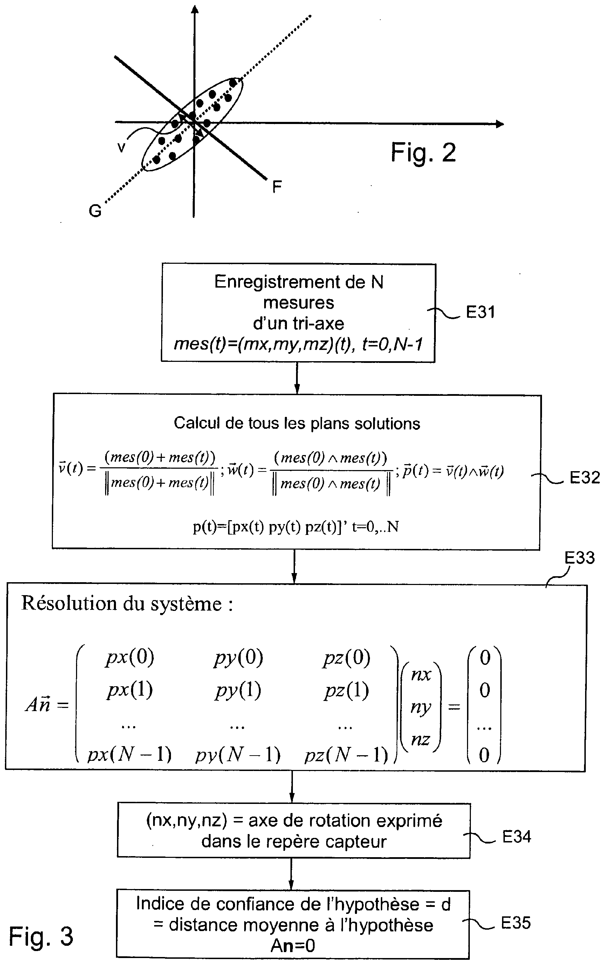

On a illustré à la figure 2, de manière simplifiée dans un espace à deux dimensions, des mesures représentées par un ensemble de points.

Bien entendu, cette représentation schématique peut être étendue à un espace à trois dimensions.

L'axe de plus faible variation F et l'axe de plus grande variation G sont illustrés sur la figure 2.

La flèche v selon la direction de l'axe de plus faible variation F correspond à l'écart type des mesures projetées sur cet axe et correspond ainsi à un indicateur de variations de l'axe de rotation. Plus cette variance v est faible, plus le mouvement observé correspondra à un mouvement de rotation autour d'un axe fixe.

On va décrire en référence à la figure 1 un procédé de détection d'un axe de rotation sensiblement invariant permettant de déterminer l'axe de plus faible variation à partir d'une analyse en composantes principales.

En pratique, une étape d'acquisition E10 de mesures physiques selon les trois axes de sensibilité du capteur est mise en oeuvre.

Les mesures physiques comprennent N échantillons à des instants différents selon les trois axes de sensibilité du capteur.

Afin de permettre la résolution du problème, N est supérieur ou égal à 3.

On enregistre ainsi N mesures $(mx, my, mz)(t)$ avec t variant de 0 à N - 1.

Une étape de constitution E11 d'une matrice M est ensuite mise en oeuvre de telle sorte que chaque ligne correspond à un échantillon temporel des mesures et que chaque colonne correspond à une composante obtenue selon les axes de sensibilité X, Y, Z du capteur.

On obtient ainsi :

$$M= \quad [mx(0) \; my(0) \; mz(0)$$
$$mx(1) \; my(1) \; mz(1)$$
$$\ldots$$
$$mx(N) \; my(N) \; mz(N)]$$

Simultanément, une étape de calcul E12 de la moyenne obtenue pour chaque composante selon les axes de sensibilité X, Y, Z est mise en oeuvre.

On calcule ainsi en pratique une moyenne des mesures obtenues sur chaque colonne de la matrice M.

Les moyennes suivantes sont déterminées :

Mean(mx), Mean(my), Mean(mz)

Une étape d'homogénéisation E13 est ensuite mise en oeuvre afin de soustraire la valeur moyenne calculée à l'étape E12 de chaque élément de la matrice M. On obtient ainsi une matrice Mc :

$$Mc= \quad [mx(0)-Mean(mx) \quad my(0)-Mean(my) \quad mz(0)-Mean(mz)$$
$$mx(1)-Mean(mx) \quad my(1)-Mean(my) \quad mz(1)-Mean(mz)$$
$$\ldots$$
$$mx(N)-Mean(mx) \quad my(N)-Mean(my) \quad mz(N)-Mean(mz)]$$

Sur la matrice obtenue Mc, il est possible de réaliser une analyse en composantes principales dans une étape d'analyse E14.

La détermination de l'axe de plus faible variation par une analyse en composante principale est connue et n'a pas besoin d'être décrite en détail ici. On pourra se reporter notamment à la description de cette technique dans le recueil Jolliffe I.T., Principal component analysis, 2nde édition, Springer 2002 ou encore Jackson J. E., A User's Guide to Principal Components, John Wiley and Sons, 1991, p. 4-25.

Dans son principe, cette analyse en composantes principales revient à rechercher une matrice unitaire V de dimensions 3 x 3, dans laquelle chaque vecteur colonne est de norme unitaire et orthogonal aux autres vecteurs colonnes, une matrice diagonale ordonnée SIGMA de taille 3 x 3 avec des éléments positifs de la diagonale en ordre décroissant et une matrice unitaire U de dimensions N x N telle que :

$$Mc = U \; SIGMA \; V^T$$

Ces matrices peuvent être obtenues en réalisant la décomposition en valeurs singulières de la matrice Mc ou la décomposition en valeurs propres et vecteurs propres de la matrice $S = (N - 1) \; Mc^T Mc$, de telle manière que $S V = V \; LAMBDA$ où LAMBDA est une matrice diagonale qui contient les valeurs propres de S. On a de plus $LAMBDA (i, i) = (N-1)^{-1} \; SIGMA (i, i)^2$.

Les colonnes de la matrice unitaire V sont appelées axes factoriels et l'axe de rotation recherché exprimé dans le repère associé aux capteurs, correspondant à l'axe de projection sur lequel les mesures ont la plus faible variance, correspond à la troisième colonne de la matrice unitaire V.

La valeur LAMBDA (3,3) sur la troisième colonne de la matrice diagonale LAMBDA correspond à la variance associée à l'axe ainsi déterminé.

Il est ainsi possible d'estimer l'axe de rotation sensiblement invariant dans une étape d'estimation E15 à partir de l'axe factoriel de la troisième colonne de la matrice unitaire V, l'axe de rotation étant ainsi exprimé dans le repère associé aux capteurs. Cet axe de rotation sera noté dans la suite par ses coordonnées dans le repère du capteur de la manière suivante :

(nx, ny, nz)

Afin de valider l'axe de rotation ainsi estimé, le procédé de détection comporte en outre une étape de calcul E16 d'un indicateur des variations de l'axe de rotation.

On calcule ainsi la valeur d de telle sorte que :

$$d = \frac{\text{LAMBDA}\ (3,3)}{\text{LAMBDA}\ (1,1) + \text{LAMBDA}\ (2,2) + \text{LAMBDA}\ (3,3)}$$

Cette valeur d de l'indicateur donne une indication sur la validation de l'axe de rotation estimé comme axe de rotation invariant du mouvement.

Plus la valeur d est petite, plus l'hypothèse selon laquelle le mouvement du mobile est réalisé autour d'un axe de rotation constant ou sensiblement invariant est valable.

En pratique, on compare la valeur de LAMBDA (3,3) de l'indicateur avec la valeur du bruit des mesures physiques obtenues par le capteur. Lorsque la valeur de LAMBDA (3,3) de l'indicateur est sensiblement égale ou du même ordre de grandeur que la valeur du bruit, l'axe de rotation estimé (nx, ny, nz) est validé comme axe de rotation sensiblement invariant du mouvement du mobile.

D'autre part, il faut que les variations selon les autres axes soient importantes : LAMBDA (3,3) + LAMBDA (2,2) + LAMBDA (1,1) doit être grand ce qui garantit que le mouvement réalisé est de suffisamment grande amplitude.

En pratique, si d est proche de 1/3, les trois axes ont des variations équivalentes et l'on ne peut rien conclure. On ne conservera l'hypothèse "axe de rotation constant" que si d<seuil que l'on prendra égal à 0.1 par exemple.

Un autre indicateur peut être d*= LAMBDA (3,3) / LAMBDA (2,2) qui devra être petit devant 1. On ne conservera l'hypothèse "axe de rotation constant" que si d*<seuil que l'on prendra égal à 0.1 par exemple.

D'autres méthodes permettant d'estimer l'axe de plus faible variation dans les mesures obtenues par un capteur inertiel ou magnétique peuvent également être mises en oeuvre.

A titre d'exemple, la méthode des moindres carrés peut également être utilisée.

Dans son principe, la méthode des moindres carrés revient à estimer l'axe n sur lequel la projection des mesures M est constante. Cet axe n peut être calculé par la formule suivante :

$$n = pinv(M)O$$

où

pinv(M) est l'inverse généralisé de la matrice M telle que constituée à l'étape E11 décrite en relation à la figure 1, n est un vecteur à trois composantes en colonne n = $(nx, ny, nz)^T$, et O est un vecteur de N lignes et 1 colonne dont tous les éléments sont égaux à 1.

On normalise le vecteur n pour obtenir le vecteur directeur de la normale au plan.

On notera de manière générale que le procédé de détection d'un axe de rotation décrit précédemment peut être mis en oeuvre pour différents types de capteurs inertiels ou magnétiques et par exemple un accéléromètre à trois axes de sensibilité ou un magnétomètre à trois axes de sensibilité ou encore un gyromètre à trois axes de sensibilité.

Toutefois, dans le cas d'un capteur inertiel du type gyromètre, l'axe de rotation estimé correspond non pas à l'axe de plus faible variation, mais au contraire à l'axe de plus grande variation des mesures. L'indicateur des variations de l'axe de rotation utilisé correspond alors à la variance ou l'écart-type, calculé sur les deux axes orthogonaux à l'axe de rotation estimé.

Bien entendu, un seul capteur inertiel ou magnétique permet à partir des mesures physiques en sortie de ce capteur de détecter l'axe de rotation constant sous réserve que la valeur de l'indicateur des variations de l'axe de rotation soit sensiblement égale à la valeur du bruit des mesures physiques associées.

En particulier, lorsque le capteur est un accéléromètre, si l'écart-type des mesures projetées sur l'axe de rotation estimé est sensiblement égal au bruit du capteur, l'axe de rotation estimé est validé comme axe de rotation sensiblement invariant du mouvement et le mouvement est en outre considéré comme planaire.

En pratique, lorsque le mobile est équipé de plusieurs capteurs, et par exemple d'au moins un magnétomètre à trois axes de sensibilité et d'un accéléromètre à trois axes de sensibilité, il est possible d'estimer l'axe de rotation selon le procédé décrit précédemment dans l'espace des mesures physiques associées à chacun des capteurs, c'est-à-dire au magnétomètre et à l'accéléromètre.

On valide ensuite comme axe de rotation sensiblement invariant du mouvement une combinaison linéaire des axes de rotation estimés pour les mesures physiques du magnétomètre et de l'accéléromètre.

Les coefficients de la combinaison linéaire sont compris non strictement entre 0 et 1 et leur somme est égale 1.

Ces coefficients sont fonction des valeurs des indicateurs des variations des axes de rotation du magnétomètre et/ou de l'accéléromètre.

Lorsque l'un des coefficients de la combinaison est égal à 0, cela revient à utiliser l'un ou l'autre capteur (magnétomètre ou accéléromètre) permettant d'obtenir l'estimation la plus fiable de l'axe de rotation sensiblement invariant.

Il est ainsi possible de s'affranchir du cas dans lequel la rotation a lieu autour de l'axe du champ physique considéré et associé au capteur, c'est-à-dire le champ de gravité pour les accéléromètres et le champ magnétique terrestre pour les magnétomètres.

En effet, dans ce cas, l'estimation de l'axe de rotation sera très mauvaise pour le capteur considéré et le procédé de détection validera automatiquement l'axe de rotation estimé selon l'autre capteur comme axe de rotation sensiblement invariant du mouvement.

A titre d'exemple, dans le cas d'un mouvement autour d'un axe vertical (ou dans un plan horizontal), comme celui d'une souris sur un bureau, l'utilisation d'un accéléromètre comme capteur inertiel dans le procédé de détection ne sera pas satisfaisant. Au contraire, l'utilisation d'un magnétomètre pourra permettre d'estimer l'axe de rotation vertical du mouvement.

On va décrire par ailleurs en référence à la figure 3, un second mode de réalisation de l'invention dans lequel le procédé de détection d'un axe de rotation est mis en oeuvre à partir d'une étape de calcul vectoriel réalisé sur les mesures physiques obtenues sur les trois axes de sensibilité du capteur.

Dans son principe, chaque mesure est considérée comme un vecteur dans un espace à trois dimensions.

L'axe de rotation constant dans l'espace des mesures est déterminé en réalisant l'intersection de plusieurs plans, chaque plan correspondant à l'ensemble des axes de rotation possibles permettant de passer d'un vecteur à un autre. En pratique, les vecteurs sont regroupés et analysés deux à deux afin de déterminer chaque plan.

La distance entre les plans au niveau de l'intersection est un indicateur des variations de l'axe de rotation.

Comme dans le mode de réalisation précédent, en calculant cet indicateur et en comparant la valeur de cet indicateur avec la valeur du bruit des mesures physiques du capteur, il est possible de valider l'axe de rotation estimé, c'est-à-dire l'axe correspondant à l'intersection des plans, comme axe de rotation sensiblement invariant du mouvement lorsque la valeur de l'indicateur est sensiblement égale à la valeur du bruit des mesures physiques du capteur.

En pratique, une étape d'enregistrement E31 permet d'acquérir des mesures physiques selon les trois axes de sensibilité du capteur.

On obtient ainsi N mesures selon les trois axes de sensibilité :

$$mes(t) = (mx, my, mz)(t), t = 0, ..., N - 1$$

Une étape de calcul E32 de tous les plans solution P(t) est ensuite mise en oeuvre de la manière suivante.

On définit, pour chaque instant t, les trois vecteurs suivants :

$$\vec{v}(t) = \frac{(mes(0) + mes(t))}{\left\| mes(0) + mes(t) \right\|} \; ; \; \vec{w}(t) = \frac{(mes(0) \wedge mes(t))}{\left\| mes(0) \wedge mes(t) \right\|} \; ; \; \vec{p}(t) = \vec{v}(t) \wedge \vec{w}(t) = \begin{vmatrix} px(t) \\ py(t) \\ pz(t) \end{vmatrix}$$

En pratique, à chaque instant, l'axe de la rotation que le mobile a effectuée entre l'instant t = 0 et l'instant t appartient nécessairement au plan défini par les vecteurs $\vec{v}(t)$ et $\vec{w}(t)$, ce qui correspond encore à l'équation d'un plan $\vec{p}(t).\vec{n} = 0$

Ainsi, s'il existe un axe de rotation constant dans le mouvement du mobile observé, alors il correspond à l'intersection de tous les plans définis par les vecteurs $\vec{v}(t)$ et $\vec{w}(t)$, avec t variant de 0 à N - 1.

A contrario, si l'intersection existe, on peut considérer que l'hypothèse selon laquelle le mouvement est réalisé autour d'un axe de rotation constant est validée et que cet axe de rotation correspond à l'intersection des plans.

En pratique, une étape de résolution E33 du système est mise en oeuvre à partir de la construction d'une matrice A dont les lignes correspondent aux N échantillons et les colonnes aux trois composantes du vecteur $\vec{p}$ :

$$A = \begin{pmatrix} px(0) & py(0) & pz(0) \\ \cdots & & \cdots \\ px(N-1) & py(N-1) & pz(N-1) \end{pmatrix}$$

La recherche de l'axe de rotation $\vec{n}$ (nx, ny, nz) revient à résoudre l'équation $A\vec{n} = 0$, c'est-à-dire :

$$A\vec{n} = \begin{pmatrix} px(0) & py(0) & pz(0) \\ px(1) & py(1) & pz(1) \\ \cdots & \cdots & \cdots \\ px(N-1) & py(N-1) & pz(N-1) \end{pmatrix} \begin{pmatrix} nx \\ ny \\ nz \end{pmatrix} = \begin{pmatrix} 0 \\ 0 \\ \cdots \\ 0 \end{pmatrix}$$

Les coordonnées du vecteur $\vec{n}$ peuvent être obtenues en réalisant la décomposition en valeurs singulières de la matrice A comme décrit précédemment de telle sorte que $A = USV^T$.

De la même manière que pour l'analyse en composantes principales, les termes diagonaux sont classés par ordre décroissant.

L'axe de rotation $\vec{n}$ correspond alors à la troisième colonne de la matrice unitaire V.

Les valeurs de la diagonale de la matrice S permettent comme précédemment de calculer la valeur d d'un indicateur des variations de l'axe de rotation, qui correspond à la distance moyenne à l'hypothèse $A\vec{n} = 0$.

On observera que la résolution du système peut être facilitée lorsque le nombre d'échantillons N est important, en réalisant d'abord une triangularisation orthogonale de la matrice A et en appliquant ensuite une décomposition en valeurs singulières directement à la matrice triangularisée.

Par ailleurs, l'estimation du vecteur n peut être améliorée, notamment en supprimant de la matrice les mesures pour lesquelles mes(t) et mes(0) sont proches.

En effet, lorsque la distance entre les vecteurs est faible, l'orientation du vecteur $\vec{w}$ est fortement perturbée par le bruit des mesures, de telle sorte que l'estimation générale du vecteur $\vec{n}$ par la matrice A se trouve également perturbée.

Il est possible, afin de limiter cet effet, de supprimer de la matrice A les lignes correspondant à des observations telles que la distance entre les vecteurs mes(t) et mes(0) soit inférieure à une valeur seuil s où ce seuil s est prédéterminé en fonction du bruit des mesures obtenues en sortie du capteur associé.

On notera que ce procédé de détermination par analyse vectorielle peut être appliqué sur les mesures réalisées à partir de magnétomètres en l'absence de perturbations magnétiques ou sur des mesures obtenues en sortie d'accéléromètres en l'absence d'accélération.

En revanche, ce type de calculs vectoriels sur les mesures physiques n'est pas applicable à un vecteur inertiel de type gyromètre.

Dans les modes de réalisation décrits précédemment, il est nécessaire de disposer d'un nombre N d'échantillons au moins égal à 3 de manière à détecter l'axe de rotation.

En outre, il faut que la distance entre les mesures soit plus importante que le bruit des mesures.

Ainsi, pour un magnétomètre dont le bruit est sensiblement égal à 0,1, il est nécessaire d'avoir une distance entre les mesures d'au moins 0,3.

En pratique, cela signifie que le procédé de détection pour déterminer l'axe de rotation est particulièrement bien adapté lorsque les N échantillons permettent de couvrir des déplacements en rotation suffisamment importants autour de cet axe de rotation.

Le procédé de détection décrit précédemment peut en outre être complété par une étape de transformation des coordonnées de l'axe de rotation estimé n = (nx,ny,nz) dans le repère de mesure du capteur en coordonnées dans un repère géophysique.

Le repère géophysique peut être typiquement le repère NED (correspondant à l'acronyme « *North-East-Down* »).

A titre d'exemple, les coordonnées de l'axe de rotation dans le repère géophysique NED peuvent être calculées à partir des mesures projetées sur l'axe de rotation exprimées dans le repère du capteur de la façon suivante.

Dans le repère géophysique NED, le champ gravitationnel s'écrit g = (0,0,1) et le champ magnétique b = (bx,0,bz).

En effet, le champ magnétique est variable en fonction du lieu de mesure, les valeurs bx et bz étant la référence du champ magnétique terrestre à l'endroit où on a effectué la mesure.

$$bz \simeq \frac{\sqrt{3}}{2}.$$

A titre d'exemple, en France, $bx \simeq 1/2$ et $\qquad$ 2

On recherche ainsi les coordonnées $nz_{NED}$, $nx_{NED}$, $ny_{NED}$ du vecteur dans le repère géophysique NED.

Si on exprime les mesures accélérométriques dans le nouveau repère en faisant le produit matriciel newmesacc = $mes_a V$, on doit observer que la troisième composante est quasi constante. Elle reflète l'inclinaison du plan dans le repère inertiel et correspond à la coordonnée $nz_{NED}$.

$$nz_{NED} = \text{mean (newmesaccz)}$$

De même, si on exprime les mesures accélérométriques dans le nouveau repère en faisant le produit matriciel newmesmag = $Mes_b V$, on doit observer que la troisième composante est quasi constante. Elle est le reflet conjoint de l'inclinaison du plan et de son orientation par rapport au Nord magnétique local dans le repère inertiel et correspond à la coordonnée z.

On a la relation     tmp=mean (newmesmagz)

tmp=0.5 $nx_{NED}$ + 0.86 $nz_{NED}$

ou encore     $nx_{NED}$ = 2*tmp-sqrt(3)*$nz_{NED}$

Enfin la coordonnée $ny_{NED}$ peut être déterminée en recherchant le vecteur normé avec les coordonnées déterminées précédemment :

$$ny_{NED} = \pm\sqrt{1 - nx_{NED}^2 - nz_{NED}^2}$$

A partir de la détection d'un axe de rotation sensiblement invariant selon le procédé décrit précédemment, il est possible d'estimer le mouvement complet d'un mobile équipé d'au moins un capteur inertiel ou magnétique à trois axes de sensibilité.

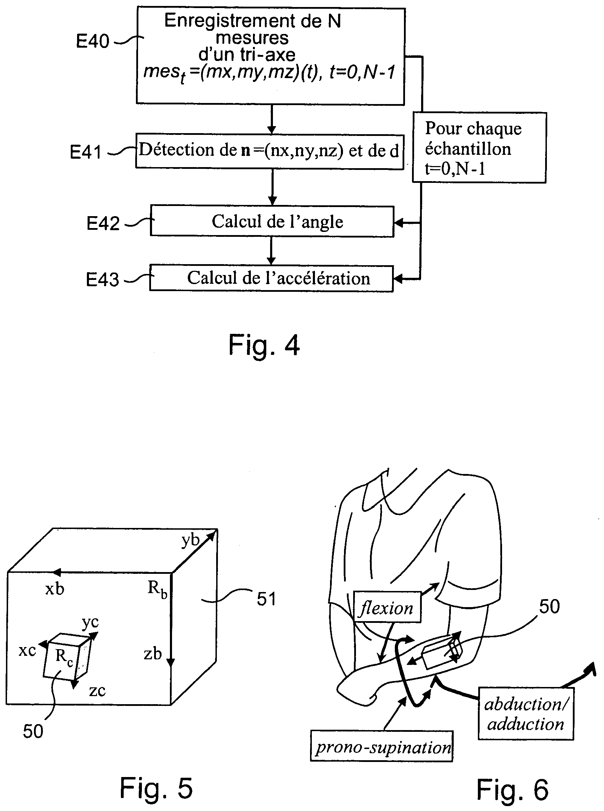

Le procédé d'estimation du mouvement d'un mobile est illustré en particulier à la figure 4.

Après une étape d'enregistrement E40 de N mesures, correspondant aux étapes d'enregistrement E10 ou E31 des figures 1 et 3, une étape de détection E41 est mise en oeuvre comme décrit précédemment afin de détecter un axe de rotation représenté par ses coordonnées n = (nx,ny,nz).

Il est alors possible successivement de calculer l'angle de rotation dans une étape de calcul E42 puis l'accélération du mobile dans une étape de calcul E43, et ceci pour chaque échantillon de mesure à des instants t compris entre 0 et N - 1 et pour les échantillons suivants si l'axe de rotation ne varie plus.

En pratique, l'étape de calcul E42 de l'angle de rotation peut être mise en oeuvre de la façon suivante.

Une fois l'axe de rotation estimé, on peut calculer à chaque instant l'angle $\theta$ de la rotation effectuée par un calcul analytique :

$$\theta = +2\arcsin\frac{\|mes(0) - mes(t)\|}{\sqrt{\|mes(0) - mes(t)\|^2 + \|mes(0) + mes(t)\|^2(\vec{w}.\vec{n})^2}}$$

Le signe de $\theta$ correspond à celui qui permet de reconstruire au mieux la mesure mes(t).

Une deuxième méthode pourrait être utilisée en estimant $\theta$ par optimisation :

$$\min_\theta\left(\|mes(t) - M(\vec{n},\theta)mes(0)\|^2\right)$$

où $M(\vec{n},\theta)$ est la matrice de rotation d'axe n et d'angle $\theta$.

On peut également se servir de la matrice V obtenue par analyse en composantes principales telle que décrite en référence à l'étape E14 de la figure 1, pour faire un changement de repère des données.

En reprenant les notations précédentes, les mesures dans le nouveau repère sont données par M'=VM. Dans ce repère la troisième composante sera constante.

Pour chaque instant temporel (ligne de la matrice M' de dimensions N lignes et 3 colonnes), l'angle de la rotation est l'angle formé par les 2 vecteurs 2D (leurs coordonnées correspondant aux deux premières colonnes).

En pratique, on cherche l'angle entre deux vecteurs correspondant à deux lignes de la matrice qui correspond à la rotation effectuée entre ces deux instants.

Pour cela, on peut par exemple exprimer ces vecteurs en coordonnées polaires (longueur, orientation) puis faire la différence de leurs orientations. On peut également utiliser leur produit scalaire.

Dans le cas d'un mouvement accéléré, une fois l'orientation du mobile estimée à chaque instant t à l'aide d'un magnétomètre ou d'un goniomètre, on peut estimer l'accélération du mobile à l'aide d'un accéléromètre.

Sachant que les mesures des accéléromètres s'écrivent:

$$mes(t)=M(n,\theta)(g-a)= M(n,\theta)(mes(0)-a)$$

où M(n,θ) est la matrice de rotation permettant de passer du repère fixe au repère mobile,
θ l'angle de la rotation,
a l'accélération recherchée, et
g le champ gravitationnel égal à mes(0) ds le repère de référence pris à l'instant 0.

On peut estimer l'accélération a du mobile dans le repère fixe par :

$$\vec{\tilde{a}} = -M(\vec{n},\theta)^T \, mes(t) + mes(0)$$

où a et mes(0) sont exprimés dans le même repère fixe.

Dans le cas particulier d'un mouvement planaire, l'accélération a doit être contenue dans le plan orthogonal à n. Le problème à résoudre revient alors à une optimisation avec contraintes :

$$\min_{\tilde{a}} \left( \left\| mes(t) - M(\vec{n},\theta)(mes(0) - \vec{\tilde{a}}) \right\|^2 \right) \quad avec \quad \vec{n} \cdot \vec{\tilde{a}} = 0$$

C'est un problème classique de moindres carrés avec contraintes linéaires que l'on peut résoudre de nouveau par des techniques de factorisation orthogonale.

Soit H la matrice de rotation telle que l'axe x devient l'axe n (ce qui s'écrit Hn=[100]'). Pour trouver H, on peut réaliser la décomposition orthogonale triangulaire de n. Soit H̃ = H(:,2: 3) la matrice constituée des deux dernières colonnes de H.

Alors, on obtient l'accélération du mobile par :

$$\vec{\tilde{a}}_t = \widetilde{H}\widetilde{H}^T (-M(\vec{n},\theta)mes(t) + mes(0))$$

A titre d'exemple non limitatif, le mouvement observé peut-être la marche. On place par exemple le capteur sur le plateau tibial. L'alignement précis du capteur n'est plus nécessaire si on s'intéresse uniquement à l'angle décrit dans le plan sagittal de marche.

On peut appliquer la méthode d'estimation de mouvement décrite précédemment sur chaque segment temporel correspondant à une foulée ou un pas.

Par ailleurs, grâce au procédé de détection d'un axe invariant décrit précédemment, il est possible par la mise en oeuvre de ce procédé, de réaliser la calibration d'un système de capteurs, c'est-à-dire de déterminer une matrice de rotation adaptée à transformer un repère de mesures du capteur en un repère prédéterminé solidaire d'un mobile portant le capteur.

Comme illustré à la figure 5, lorsque le capteur 50 est intégré à un boîtier 51, les axes xb, yb, zb du repère prédéterminé Rb sont définis par des arêtes du boîtier 51.

Le procédé de calibration qui va être décrit ci-après permet de déterminer la matrice de rotation permettant de passer du repère Rc associé au capteur 50 au repère prédéterminé solidaire du boîtier Rb.

De même, comme illustré à la figure 6, le capteur 50 peut être positionné sur une partie anatomique, et par exemple l'avant-bras d'un individu.

Le repère prédéterminé correspond alors à un repère anatomique défini par trois axes de rotation anatomique de cette partie anatomique.

Comme bien illustré à la figure 6, les axes du repère anatomique peuvent être définis par les axes de rotation respectivement d'un mouvement de flexion de l'avant-bras, d'un mouvement de prono-supination de l'avant-bras et d'un mouvement d'abduction/adduction de l'avant-bras.

Dans son principe, le procédé de calibration consiste à réaliser successivement des rotations selon les trois axes du repère prédéterminé Rb.

En cherchant ensuite pour chacun des trois mouvements, les coordonnées de l'axe de rotation correspondant dans le repère du capteur Rc, il est possible d'élaborer une matrice de rotation dans laquelle les coordonnées de chacun des vecteurs de rotation trouvés correspond à une colonne de la matrice de rotation qui permet de passer du repère de capteur Rc au repère prédéterminé solidaire du mobile Rb.

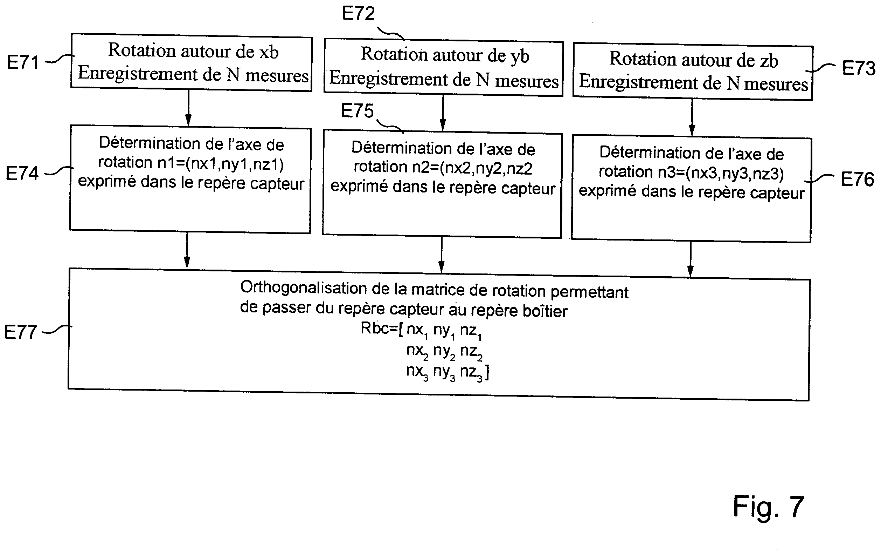

Comme illustré à la figure 7, le procédé de calibration comporte notamment des étapes d'acquisition E71, E72, E73 de mesures physiques par le capteur, correspondant respectivement à chaque rotation autour des axes xb, yb, zb du repère Rb.

Pour chaque mouvement de rotation, N échantillons de mesures sont enregistrés comme décrit précédemment en référence par exemple à l'étape d'enregistrement E10 décrite à la figure 1.

A partir de chaque enregistrement de N mesures, une étape de détermination E74, E75, E76 est mise en oeuvre afin de déterminer les coordonnées des trois axes de rotation n1, n2, n3 correspondant aux axes du repère prédéterminé Rb dans le repère des mesures physiques Rc du capteur. Ces étapes de détermination de l'axe de rotation E74, E75, E76 sont mises en oeuvre comme décrit précédemment par le procédé de détection d'un axe de rotation sensiblement invariant.

Une étape d'élaboration E77 d'une matrice Rbc est ensuite mise en oeuvre à partir des coordonnées des axes de rotation déterminées dans le repère des mesures physiques Rc :

$$Rbc=\begin{bmatrix} nx1 & ny1 & nz1 \\ nx2 & ny2 & nz2 \\ nx3 & ny3 & nz3 \end{bmatrix}$$

Lors de l'étape d'élaboration E77 de la matrice Rbc, il est également possible d'orthogonaliser la matrice ainsi formée par les trois vecteurs n1, n2, n3 de manière à la rendre unitaire. La matrice Rbc correspond alors à une matrice de rotation permettant d'exprimer les mesures du repère capteur Rc dans le repère prédéterminé Rb solidaire du mobile.

Ainsi, les mesures dans le nouveau repère Rb pourront être exprimées de la manière suivante :

$$mes_b = Rbc\ mes_c$$

dans lequel $mes_b$ correspond aux mesures dans le repère associé au boîtier et $mes_c$ correspond aux mesures dans le repère associé au capteur.

On notera que dans le procédé décrit précédemment, seules deux des trois rotations autour des axes du repère prédéterminé Rb peuvent être réalisées, les coordonnées du troisième axe de rotation étant alors obtenues en orhogonalisant la matrice formée par les cordonnées des deux premiers axes de rotation déterminés par le procédé de détection conforme à l'invention.

D'autre part, le repère initial peut ne pas être orthogonal (les axes ne sont pas orthogonaux les uns par rapport aux autres). Si le repère final est orthogonal, alors la matrice Rbc qui fait le changement de repère permet donc de passer des données exprimées dans un repère non orthogonal à des données exprimées dans un repère orthogonal. Il faut obligatoirement pour cela réaliser les trois rotations et ne pas orthogonaliser la matrice Rbc obtenue.

On peut aussi choisir de passer par un repère orthogonal intermédiaire, puis de faire un changement de repère entre deux repères orthogonaux (et dans ce cas, seulement deux rotations sur les trois sont nécessaires).

Ainsi, grâce au procédé de détection d'un axe de rotation sensiblement constant conforme à l'invention, il est possible de diminuer le nombre de degrés de liberté dans le mouvement d'un mobile observé et ainsi de faciliter la résolution des systèmes permettant de déterminer les paramètres de ce mouvement.

Ce procédé permet d'utiliser une centrale inertielle comprenant moins de capteurs inertiels ou magnétiques que dans les procédés de l'art antérieur et en particulier sans gyromètre.

Le procédé d'estimation d'un mouvement mettant en oeuvre la détection d'un axe de rotation constant est particulièrement bien adapté au cas dans lequel l'axe de rotation est lentement variable sous réserve d'enregistrer les échantillons N de mesure sur une fenêtre temporelle de petite taille. On peut ainsi réaliser, sur chaque fenêtre temporelle, la détermination d'un axe de rotation sensiblement invariant sur cette fenêtre temporelle dès lors qu'il existe des mesures suffisamment éloignées les unes des autres sur cette fenêtre temporelle, c'est-à-dire ayant une distance suffisante par rapport au bruit des mesures du capteur.

Ainsi, pour chaque fenêtre temporelle de petite taille, on calcule la plus grande distance séparant les vecteurs de mesure pris deux à deux.

Si cette distance est inférieure à un seuil prédéterminé qui dépend du bruit des capteurs, alors on agrandit la fenêtre temporelle et on réenregistre un échantillon de N mesures sur cette nouvelle fenêtre.

Sinon, si la distance maximale entre les vecteurs de mesure est supérieure au bruit des capteurs, on calcule l'axe de rotation comme indiqué précédemment en référence au procédé de détection d'un axe de rotation sensiblement invariant et l'indicateur de variations de cet axe de rotation afin de valider l'existence d'un axe de rotation constant dans le mouvement observé.

Eventuellement, l'angle et l'accélération du mouvement sur cette fenêtre temporelle peuvent également être estimés dès lors que l'on souhaite observer le mouvement d'un mobile dans le temps.

L'ensemble des opérations est ensuite réitéré sur une fenêtre temporelle décalée dans le temps.

**Revendications**

**1.** Procédé de détection d'un axe de rotation sensiblement invariant d'un mouvement d'un mobile équipé d'au moins un magnétomètre à trois axes de sensibilité et un accéléromètre à trois axes de sensibilité comprenant les étapes suivantes :

- acquisition (E10 ; E31) de mesures physiques selon les trois axes de sensibilité du magnétomètre et de l'accéléromètre, les mesures physiques comprenant au moins trois échantillons à des instants différents dudit mouvement ;
- estimation (E11-E15; E32-E34) des axes de rotation sensiblement invariants dans les espaces desdites mesures physiques respectivement dudit magnétomètre et dudit accéléromètre à partir desdits au moins trois échantillons acquis, chaque axe de rotation sensiblement invariant correspondant à l'axe pour lequel l'écart type des valeurs desdites mesures physiques projetées sur ledit axe est minimisé, l'étape d'estimation comprenant

  ■ une étape de calcul (E14) par analyse en composantes principales, la troisième composante principale correspondant aux coordonnées dudit axe de rotation sensiblement invariant dans l'espace desdites mesures physiques respectivement dudit magnétomètre et dudit accéléromètre, ou
  ■ une étape de calcul des moindres carrés appliquée à une combinaison linéaire desdites mesures physiques respectivement dudit magnétomètre et dudit accéléromètre;

- calcul (E16 ; E35) d'un indicateur (d) des variations de l'axe de rotation respectivement estimé pour l'accéléromètre et le magnétomètre ; et
- validation, comme axe de rotation sensiblement invariant dudit mouvement, d'une combinaison linéaire des axes de rotation estimés pour lesdites mesures physiques du magnétomètre et de l'accéléromètre, les coefficients de la combinaison étant compris non strictement entre 0 et 1, leur somme étant égale à 1, et étant fonction des valeurs des indicateurs des variations des axes de rotation du magnétomètre et/ou de l'accéléromètre.

**2.** Procédé de détection conforme à la revendication 1 comprenant en outre les étapes suivantes :

- comparaison (E16 ; E35) de la valeur de chaque indicateur avec une valeur de seuil prédéterminée ; et
- validation de l'axe de rotation respectivement estimé pour l'accéléromètre et le magnétomètre comme axe de rotation sensiblement invariant dudit mouvement lorsque la valeur dudit indicateur (d) est inférieure à la valeur de seuil prédéterminée, la valeur de seuil étant avantageusement égale à la valeur du bruit des mesures physiques respectivement de l'accéléromètre et du magnétomètre.

**3.** Procédé de détection conforme à l'une des revendications précédentes, où si pour l'accéléromètre et pour le magnétomètre ledit écart type est sensiblement égal à l'écart type du bruit dudit accéléromètre ou magnétomètre, l'axe de rotation estimé est validé comme axe de rotation sensiblement invariant dudit mouvement.

**4.** Procédé de détection conforme à l'une des revendications précédentes, comportant en outre respectivement pour l'accéléromètre et le magnétomètre une étape de transformation des coordonnées de l'axe de rotation estimé dans le repère de mesure dudit capteur en coordonnées dans un repère géophysique.

**5.** Procédé d'estimation d'un mouvement d'un mobile équipé d'au moins un magnétomètre à trois axes de sensibilité et un accéléromètre à trois axes de sensibilité, ledit mouvement comprenant une rotation autour d'un axe sensiblement invariant, **caractérisé en ce qu'**il comprend les étapes suivantes :

- détection (E41) d'un axe de rotation sensiblement invariant selon le procédé de détection conforme à l'une des revendications 1 à 4 ;
- détermination (E42) de l'angle de rotation dudit mobile autour dudit axe de rotation sensiblement invariant.

6. Procédé d'estimation conforme à la revendication 5 comprenant en outre une étape de détermination (E43) de l'accélération dudit mobile.

7. Dispositif de détection d'un axe de rotation sensiblement invariant d'un mouvement d'un mobile équipé d'au moins un magnétomètre à trois axes de sensibilité et un accéléromètre à trois axes de sensibilité, comprenant des moyens adaptés à mettre en oeuvre le procédé de détection conforme à l'une des revendications 1 à 4.

**Patentansprüche**

1. Verfahren zum Ermitteln einer im Wesentlichen invarianten Rotationsachse einer Bewegung eines beweglichen Körpers, der mit mindestens einem Magnetometer mit drei Empfindlichkeitsachsen und einem Beschleunigungsmesser mit drei Empfindlichkeitsachsen versehen ist, umfassend die folgenden Schritte:

   - Erfassen (E10; E31) physikalischer Messungen entlang der drei Empfindlichkeitsachsen des Magnetometers und des Beschleunigungsmessers, wobei die physikalischen Messungen zumindest drei Abtastungen zu unterschiedlichen Zeitpunkten der Bewegung umfassen;
   - Schätzen (E11 - E15; E32 - E34) der im Wesentlichen invarianten Rotationsachsen in den Räumen der physikalischen Messungen jeweils des Magnetometers und des Beschleunigungsmessers ausgehend von den zumindest drei erfassten Abtastungen, wobei jede im Wesentlichen invariante Rotationsachse der Achse entspricht, bei der die Standardabweichung der auf die Achse projizierten Werte der physikalischen Messungen minimiert ist, wobei der Schritt des Schätzens umfasst:

      • einen Schritt des Berechnens (E14) durch Hauptkomponentenanalyse, wobei die dritte Hauptkomponente den Koordinaten der im Wesentlichen invarianten Rotationsachse im Raum der physikalischen Messungen jeweils des Magnetometers und des Beschleunigungsmessers entspricht, oder
      • einen Schritt des Berechnens der kleinsten Quadrate, der auf eine lineare Kombination der physikalischen Messungen jeweils des Magnetometers und des Beschleunigungsmessers angewendet wird;

   - Berechnen (E16; E35) eines Indikators (d) der Änderungen der jeweils geschätzten Rotationsachse für den Beschleunigungsmesser und das Magnetometer; und
   - Validieren einer linearen Kombination der geschätzten Rotationsachsen für die physikalischen Messungen von Magnetometer und Beschleunigungsmesser als im Wesentlichen invariante Rotationsachse der Bewegung, wobei die Koeffizienten der Kombination nicht streng zwischen 0 und 1 liegen, ihre Summe gleich 1 ist, und sie Funktion der Werte der Indikatoren der Änderungen der Rotationsachsen des Magnetometers und/oder des Beschleunigungsmessers sind.

2. Ermittlungsverfahren nach Anspruch 1, ferner umfassend die nachfolgenden Schritte:

   - Vergleichen (E16; E35) des Werts eines jeden Indikators mit einem vorbestimmten Schwellwert; und
   - Validieren der jeweils geschätzten Rotationsachse für den Beschleunigungsmesser und das Magnetometer als im Wesentlichen invariante Rotationsachse der Bewegung dann, wenn der Wert des Indikators (d) kleiner als der vorbestimmte Schwellwert ist, wobei der Schwellenwert vorteilhaft gleich dem Rauschwert der physikalischen Messungen jeweils des Beschleunigungsmessers und des Magnetometers ist.

3. Ermittlungsverfahren nach einem der vorangehenden Ansprüche, wobei dann, wenn für den Beschleunigungsmesser und das Magnetometer die Standardabweichung im Wesentlichen gleich der Standardabweichung des Rauschens von Beschleunigungsmesser bzw. Magnetometer ist, die geschätzte Rotationsachse als im Wesentlichen invariante Rotationsachse der Bewegung validiert wird.

4. Ermittlungsverfahren nach einem der vorangehenden Ansprüche, ferner umfassend jeweils für den Beschleunigungsmesser und das Magnetometer einen Schritt der Transformation der Koordinaten der geschätzten Rotationsachse in dem Messbezugssystem des Sensors in Koordinaten in einem geophysikalischen Bezugssystem.

5. Verfahren zum Schätzen einer Bewegung eines beweglichen Körpers, der mit mindestens einem Magnetometer mit drei Empfindlichkeitsachsen und einem Beschleunigungsmesser mit drei Empfindlichkeitsachsen versehen ist, wobei die Bewegung eine Drehung um eine im Wesentlichen invariante Achse umfasst, **dadurch gekennzeichnet, dass** es die nachfolgenden Schritte umfasst:

- Ermitteln (E41) einer im Wesentlichen invarianten Rotationsachse mit dem Ermittlungsverfahren nach einem der Ansprüche 1 bis 4;
- Bestimmen (E42) des Rotationswinkels des beweglichen Körpers um die im Wesentlichen invariante Rotationsachse.

6. Schätzungsverfahren nach Anspruch 5, ferner umfassend einen Schritt des Bestimmens (E43) der Beschleunigung des beweglichen Körpers.

7. Vorrichtung zum Ermitteln einer im Wesentlichen invarianten Rotationsachse einer Bewegung eines beweglichen Körpers, der mit zumindest einem Magnetometer mit drei Empfindlichkeitsachsen und einem Beschleunigungsmesser mit drei Empfindlichkeitsachsen versehen ist, enthaltend Einrichtungen, die dazu geeignet sind, das Ermittlungsverfahren nach einem der Ansprüche 1 bis 4 durchzuführen.

**Claims**

1. A method of detecting a substantially invariant rotation axis of a motion of a mobile equipped with at least a magnetic sensor with three sensitive axes and an inertial sensor with three sensitive axes, the method comprising the following steps:

   - acquiring (E10; E31) physical measurements with respect to the three sensitive axes by the magnetic sensor and the inertial sensor, the physical measurements comprising at least three samples at different times during said motion;
   - estimating (E11-E15 - E32-E34) the three substantially invariant rotation axes in the physical measurement space of said magnetic sensor and said inertial sensor from said at least three acquired samples, each substantially invariant rotational axis corresponding to the axis for which the standard deviation of the values of said physical measurements projected onto said axis is minimized, said estimating step comprising
   - a step of calculating (E14) by analysis into principal components, the third principal component corresponding to the coordinates of said substantially invariant rotation axis in the physical measurements spaces, respectively of said magnetic sensor and said inertial sensor, or
   - a step of calculating least squares applied to a linear combination of said physical measurements, respectively of said magnetic sensor and of said inertial sensor;
   - calculating (E16; E35) an indicator (d) of variations of the rotation axis estimated respectively for the inertial sensor and the magnetic sensor; and
   - validating, as the substantially invariant rotation axis of said motion, the linear combination of the estimated rotational axis for said physical measurements of the magnetic sensor and the inertial sensor, the coefficients of the combination being comprised not strictly between 0 and 1, the sum being equal to 1, and being function of the values of the indicators of the variations of the rotational axis of the magnetic sensor and/or of the inertial sensor.

2. The method for detecting according to claim 1, further comprising the following steps:

   - comparing (E16; E35) the value of each indicator with a predetermined threshold value; and
   - validating the estimated substantially invariant rotation axis for the inertial sensor and the magnetic sensor as the substantially invariant rotation axis of the motion if the value of said indicator (d) is less than the predetermined threshold value, said threshold value being advantageously equal to the value of the noise affecting the physical measurements respectively from the inertial sensor and the magnetic sensor.

3. The method of detecting according to one of the preceding claims, wherein, if for the inertial sensor and for the magnetic sensor said standard deviation is substantially equal to the standard deviation of noise affecting the inertial sensor or magnetic sensor, the estimated rotation axis is validated as the substantially invariant rotation axis of the motion.

4. The method of detecting according to one of the preceding claims, further comprising, respectively for the inertial sensor and for the magnetic sensor a step of transforming coordinates of the estimated rotation axis in the measurement frame of reference of said sensor into coordinates in a geophysical frame of reference.

5. A method for estimating a motion of a mobile body equipped with at least a magnetic sensor with three sensitive axes and an inertial sensor with three sensitive axes, said motion comprising rotation about a substantially invariant axis, **characterized in that** the method comprised the following steps:

- detecting (E41) a substantially invariant rotation axis by the detection method according to one of the claims 1 to 4;
- determining (E42) a rotation angle of said mobile body about said substantially invariant rotation axis.

6. The method of estimating according to claim 5 further comprising a step of determining (E43) the acceleration of the mobile body.

7. A device for detecting a substantially invariant rotation axis of a motion of a mobile body equipped with at least a magnetic sensor with three sensitive axes and an inertial sensor with three sensitive axes, comprising means for implementing the method of detecting according to one of the claims 1 to 4.

Enregistrement de N mesures
d'un tri-axe (mx,my,mz)(t), t=0,N-1 ⌐ E10

E12

Constitution de la matrice M
N lignes, 3 colonnes
M=[mx(0) my(0) mz(0)
mx(1) my(1) mz(1)
E11 ⌐ ...
mx(N) my(N) mz(N)]

Calcul de la moyenne pour
chaque composante
Mean(mx),Mean(my),Mean(mz)

Retrait de la moyenne
Mc=[mx(0)-Mean(mx) my(0) )-Mean(my) mz(0) )-Mean(mz)
mx(1)-Mean(mx) my(1) )-Mean(my) mz(1) )-Mean(mz)
...
mx(N)-Mean(mx) my(N) )-Mean(my) mz(N) )- Mean(mz)]

E13

Analyse en composantes principales
$Mc = U \ SIGMA \ V^T$
avec SIGMA ordonnée ⌐ E14

$3^{ème}$ colonne de V = axe de rotation exprimé dans
le repère capteur = (nx,ny,nz) ⌐ E15

Valeur de LAMBDA la plus
faible = $3^{ème}$ terme diagonal de
LAMBDA = indicateur de confiance ⌐ E16

Fig. 1

## Fig. 2

**Fig. 2**

---

**Enregistrement de N mesures d'un tri-axe**
$mes(t)=(mx,my,mz)(t)$, $t=0,N-1$

— E31

**Calcul de tous les plans solutions**

$$\vec{v}(t) = \frac{(mes(0)+mes(t))}{\|mes(0)+mes(t)\|} ; \vec{w}(t) = \frac{(mes(0) \wedge mes(t))}{\|mes(0) \wedge mes(t)\|} ; \vec{p}(t) = \vec{v}(t) \wedge \vec{w}(t)$$

$$p(t)=[px(t)\ py(t)\ pz(t)]'\ t=0,..N$$

— E32

E33

**Résolution du système :**

$$A\vec{n} = \begin{pmatrix} px(0) & py(0) & pz(0) \\ px(1) & py(1) & pz(1) \\ ... & ... & ... \\ px(N-1) & py(N-1) & pz(N-1) \end{pmatrix} \begin{pmatrix} nx \\ ny \\ nz \end{pmatrix} = \begin{pmatrix} 0 \\ 0 \\ ... \\ 0 \end{pmatrix}$$

**(nx,ny,nz) = axe de rotation exprimé dans le repère capteur**

— E34

**Indice de confiance de l'hypothèse = d = distance moyenne à l'hypothèse An=0**

— E35

**Fig. 3**

Fig. 4

Fig. 5

Fig. 6

E72

E71 — Rotation autour de xb Enregistrement de N mesures | Rotation autour de yb Enregistrement de N mesures | Rotation autour de zb Enregistrement de N mesures — E73

E75

E74 — Détermination de l'axe de rotation $n1=(nx1,ny1,nz1)$ exprimé dans le repère capteur | Détermination de l'axe de rotation $n2=(nx2,ny2,nz2$ exprimé dans le repère capteur | Détermination de l'axe de rotation $n3=(nx3,ny3,nz3)$ exprimé dans le repère capteur — E76

E77 — Orthogonalisation de la matrice de rotation permettant de passer du repère capteur au repère boîtier

$$Rbc=\begin{bmatrix} nx_1 & ny_1 & nz_1 \\ nx_2 & ny_2 & nz_2 \\ nx_3 & ny_3 & nz_3 \end{bmatrix}$$

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2838185

- US 3731521 A

**Littérature non-brevet citée dans la description**

- **JOLLIFFE I.T.** Principal component analysis. Springer, 2002

- **JACKSON J. E.** A User's Guide to Principal Components. John Wiley and Sons, 1991, 4-25